# EUROPÄISCHE PATENTSCHRIFT

(11) **EP 1 259 250 B1**
(45) Veröffentlichungstag und Bekanntmachung des Hinweises auf die Patenterteilung: **25.10.2006**
(21) Anmeldenummer: 01911623.5
(22) Anmeldetag: 09.02.2001
(51) Int. Cl.: A61K 38/15, A61K 47/22, A61K 47/10, A61K 47/14, A61P 33/00, A61K 38/12

(54) **ENDOPARASITIZIDE MITTEL**
ENDOPARASITICIDAL AGENTS
AGENTS ENDOPARASITICIDES

(30) Priorität: 22.02.2000 DE 10008128
(43) Veröffentlichungstag der Anmeldung: 27.11.2002
(73) Patentinhaber: Bayer HealthCare AG, 51368 Leverkusen (DE)
(72) Erfinder: KALBE, Jochen, 42799 Leichlingen (DE); TRÄUBEL, Michael, 50733 Köln (DE); VON SAMSON-HIMMELSTJERNA, Georg, 42657 Solingen (DE); HARDER, Achim, 51109 Köln (DE); STEGEMANN, Michael, 24161 Altenholz (DE); MENCKE, Norbert, 51381 Leverkusen (DE)
(86) Internationale Anmeldenummer: PCT/EP2001/001392
(87) Internationale Veröffentlichungsnummer: WO 2001/062268

(56) Entgegenhaltungen:
- EP-A- 0 662 326
- WO-A-00/69425
- WO-A-99/42125
- DE-A- 4 317 432
- DE-A- 4 317 458
- DE-A- 19 520 275
- DE-A- 19 545 044
- DATABASE WPI Section Ch, Week 199538 Derwent Publications Ltd., London, GB; Class A96, AN 1995-292942 XP002173011 & WO 95 21624 A (NIPPON KAYAKU KK), 17. August 1995 (1995-08-17)

## Beschreibung

Die vorliegende Erfindung betrifft transdermal applizierbare Mittel, enthaltend cyclische Depsipeptide, ihre Herstellung und Verwendung zur Bekämpfung von Endoparasiten.

Ein cyclisches Depsipeptid PF1022 und seine Wirkung gegen Endoparasiten ist bekannt aus EP-A 382 173.

Weitere cyclische Depsipeptide und ihre endoparasitizide Wirkung sind Gegenstand der deutschen Patentanmeldungen DE-A 4 317 432.9; DE-A 4 317 457.4; DE-A 4 317 458.2.

In der Patentschrift US 3 004 894 werden Mittel enthaltend antibiotische Wirkstoffe zum Einsatz durch Injektion beschrieben. Diese Mittel unterscheiden sich daher grundlegend von den erfindungsgemäßen Mitteln.

Enhancer für transdermale Penetration werden in der Patentanmeldung EP-A 0 268 460 beschrieben. Diese Stoffe sind aber nicht als Lösungsmittel für Depsipetide geeignet und können daher höchstens als tensidischer Zusatz für transdermale Mittel betrachtet werden.

Die Wirkungshöhe und/oder Wirkungsdauer der vorbekannten Mittel ist jedoch, insbesondere gegen bestimmte Organismen und/oder bei niedrigen Anwendungskonzentrationen, nicht in allen Anwendungsgebieten völlig zufriedenstellend.

Wegen der vielfältigen Anforderungen an moderne Arzneimittel, beispielsweise was Wirkhöhe, Wirkdauer, Wirkspektrum, Anwendungsspektrum, Toxizität, Kombination von Wirkstoffen, Kombination mit Formulierungshilfsmitteln und wegen des möglichen Auftretens von Resistenzen kann die Entwicklung neuer Arzneimittel nie als abgeschlossen betrachtet werden, und es besteht beständig ein hoher Bedarf an neuen Mitteln, die zumindest in Teilaspekten Vorteile gegenüber den bekannten Mitteln bringen.

Um dem Tierhalter eine möglichst einfache Applikation endoparasitischer Wirkstoffe zu ermöglichen, ist es zudem wünschenswert ein transdermal applizierbares Mittel zu Verfügung zu stellen

Wie aus der Literatur bekannt ist, sind Moleküle mit Molekulargewichten >1000 u äußerst schlecht bei topischer Applikation durch die Haut zu penetrieren. Besonders schlecht penetrieren Peptide oder Proteine mit größeren Molekulargewichten (Cevc et al, Exp. Opin. Invest Drugs 1997 6, 12; Pharmazeutische Technologie, Bauer, Frömming, Führer, 1993, S. 364, Thieme Verlag; Gurny, Teubner, Dermal and Transdermal Drug Delivery, 1993, S. 131, Wissenschaftliche Verlagsgesellschaft). Die Penetration ist jedoch bei endoparasitiziden Wirkstoffen Voraussetzung, da sie gegen Endoparasiten im Magen-Darm-Trakt zur Wirkung kommen sollen.

Gegenstand der vorliegenden Erfindung sind.

Mittel, enthaltend ein Cyclodepsipeptid der Formel 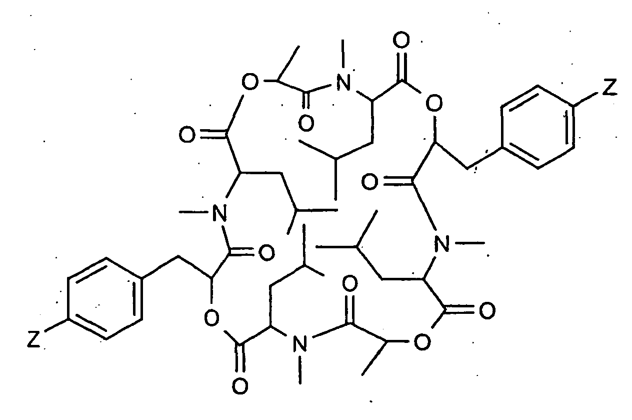 in welcher
- Z: für N-Morpholinyl, Amino, Mono- oder Dimethylamino steht,
allein oder in Mischung mit anderen Wirkstoffen, dadurch gekennzeichnet, dass die Mittel 1,2-Isopropylidenglycerin enthalten und diese Mittel zur topischen Anwendung bei Tieren geeignet sind.

Überraschenderweise wurde nun gefunden, dass die zuvor genannten Depsipeptide (mit Molgewichten >1000 u), wenn sie in Form der erfindungsgemäßen Mitteln topisch appliziert werden, bei Tieren, wie z.B. bei Hunden oder Katzen, vollständige pharmazeutische Wirksamkeit zeigen.

Weiterhin war es überraschend, dass die Depsipeptide in den erfindungsgemäßen Mitteln langanhaltende Stabilität zeigen, während in bekannten topischen Formulierungen (wie z.B. in EP A 0 682 869 beschrieben) ein äußerst schneller Abbau erfolgt.

Außerdem zeigen die erfindungsgemäßen Mittel im Gegensatz zu den bekannten Mitteln vollständige biologische Wirksamkeit.

Gegenstand der vorliegenden Erfindung ist ferner die Herstellung der erfindungsgemäßen topical applizierbaren endoparasitiziden Mittel.

Die Verbindungen der folgenden Formel 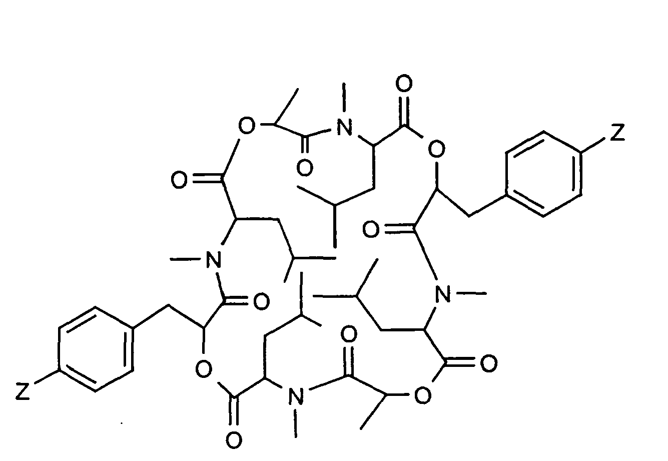 in welcher
- Z: für N-Morpholinyl, Amino, Mono- oder Dimethylamino steht,
sind aus WO93/19053 bekannt.

Von diesen wiederum besonders bevorzugt ist das Bis-Morpholino-Derivat (Z = N-Morpholinyl).

Die erfindungsgemäßen Mittel eignen sich bei günstiger Warmblütertoxizität zur Bekämpfung von pathogenen Endoparasiten die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so dass durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen insbesondere:

Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp-, Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp..

Aus der Ordnung der Enoplida z.B.: Trichuris spp., Capillaria spp., Trichomosoides spp., Trichinella spp..

Aus der Ordnung der Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp.,

Globocephalus spp., Syngamus spp., Cyathostoma spp., Metastrongylus spp., Dictyocaulus spp., Muellerius spp., protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp.

Aus der Ordnung der Oxyurida z.B.: Oxyuris spp., Enterobius spp., Passalurus spp., Syphacia spp., Aspiculuris spp., Heterakis spp.

Aus der Ordnung der Ascaridia z.B.: Ascaris spp., Toxascaris spp., Toxocara spp., Parascaris spp., Anisakis spp., Ascaridia spp.

Aus der Ordnung der Spirurida z.B.: Gnathostoma spp., Physaloptera spp., Thelazia spp., Gongylonema spp., Habronema spp., Parabronema spp., Draschia spp., Dracunculus spp.

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp.

Aus der Ordnung der Gigantorhynchida z.B.: Filicollis spp., Moniliformis spp., Macracanthorhynchus spp., Prosthenorchis spp.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Strauße, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Als Lösungsmittel kommen alle organischen Lösungsmittel, beispielsweise Ethanol, Diethylenglykolmonoethylether, Dipropylenglykolmonomethylether, Benzylbenzoat, Milchsäurebutylester, 1,2-Isopropylidenglycerin, Benzylalkohol und Propylenglykoldiacetat, bevorzugt Benzylbenzoat, Milchsäurebutylester, 1,2-Isopropylidenglycerin, Benzylalkohol und Propylenglykoldiacetat, besonders bevorzugt 1,2-Isopropylidenglycerin, Benzylalkohol und Propylenglykoldiacetat, einzeln oder als Gemische in Frage.

Die erfindungsgemäßen Mittel enthalten Lösungsmittel oder Lösungsmittelgemische, in Mengen von 95 Gew.-% bis 50 Gew.-%, bevorzugt 95 Gew.-% bis 70 Gew.-% sowie besonders bevorzugt 95 Gew.-% bis 80 Gew.-%.

Bevorzugt sind erfindungsgemäße Mittel, die mindestens 60 Gew.-% (bezogen auf das Gesamtgewicht des fertigen Mittels), bevorzugt mindestens 65 Gew.-%, an 1,2-Isopropylidenglycerin enthalten. Besonders bevorzugt enthalten diese Mittel als weiteres Lösungsmittel Benzylalkohol in Anteilen bis zu 40 Gew.-%, bevorzugt 10 bis 30 Gew.-%. Dabei werden die Anteile der Lösungsmittel selbstverständlich so gewählt, dass sie sich mit dem Wirkstoff und den gegebenenfalls verwendeten Hilfsstoffen zu 100 Gew.-% ergänzen.

Es kann vorteilhaft sein, den erfindungsgemäßen Mitteln weitere in der Veterinärmedizin übliche Hilfsstoffe, wie z.B. Verdickungsmittel zuzufügen. Beispiele für Verdickungsmittel sind: Anorganische Verdickungsmittel wie Bentonite, kolloidale Kieselsäure, Aluminiummonostearat, organische Verdickungsmittel wie Cellulosederivate (insbesondere Hydroxypropylcellulose), Polyvinylalkohole und deren Copolymere, Acrylate und Metacrylate.

Weiterhin können als Hilfsmittel Konservierungsstoffe, insbesondere Oxidationsstabilisatoren eingesetzt werden. Beispiele sind Butylhydroxyanisol (BHA), Butylhydroxytoluol (BHT) und Ascorbinsäure.

Die Wirkstoffe können in den erfindungsgemäßen Mitteln auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenyl-imidazothiazol, Benzimidazolcarbamate wie Febantel ferner Pyrantel, Praziquantel und Ivermectin.

Anwendungsfertige Zubereitungen enthalten die Wirkstoffe in Konzentrationen von 0,0001 - 25 Gewichtsprozent, bevorzugt von 0,1-20 Gewichtsprozent.

Die Mittel werden durch Mischen der Komponenten in den entsprechenden Mengen in geeigneten Geräten hergestellt.

Im allgemeinen hat es sich als vorteilhaft erwiesen, Mengen der erfindungsgemäßen Mischung von etwa 1 bis etwa 100 mg Wirkstoff je kg Körpergewicht pro Tag zur Erzielung wirksamer Ergebnisse zu verabreichen. Bevorzugt sind 1 bis 10 mg Wirkstoff je kg Körpergewicht.

Die folgenden Beispiele erläutern die Erfindung.

### Beispiel 1

5 Gew.-Teile Depsipeptid werden in 66,5 Gew.-Teilen Isopropylidenglycerin und 28,5 Gew.-Teilen Benzylalkohol unter Rühren gelöst. Es entsteht eine farblose klare Lösung.

### Beispiel 2 (kein Beispiel der Erfindung)

5 Gew.-Teile Depsipeptid werden in 66,5 Gew.-Teilen Propylenglykoldiacetat und 28,5 Gew.-Teilen Benzylalkohol unter Rühren gelöst. Es entsteht eine farblose klare Lösung.

### Beispiel 3

Zu einer Lösung aus Beispiel 1 werden zusätzlich 2 Gew.-Teile Hydroxypropylcellulose gelöst.

### Beispiel 4

Die Lösungen aus Beispiel 1 oder 2 werden in einer Dosierung von 5 mg Depsipeptid pro kg Körpergewicht auf das Rückenfell der mit Parasiten infizierten Tiere gegeben. Nach zwei bis vier Tagen sind die Tiere parasitenfrei.

| **Tier** | **Parasit** | **Wirkung** | **Anzahl behandelter Tiere** | **Anzahl von Parasiten befreiter Tiere** |
|---|---|---|---|---|
| 2 Hunde | Toxocara canis | 3/3 | 2 | 2 |
| 2 Katzen | Toxocara cati | 3/3 | 2 | 2 |
| 2 Hunde | Ancylostoma caninum | 3/3 | 2 | 2 |

### Beispiel 5

Die Lösung aus Beispiel 3 wird in einer Dosierung von 5 mg Wirkstoff/kg Körpergewicht mit Cooperia oncophara infizierten Rindern auf den Rücken aufgetragen. Die Reduktion der Wurminfektion beträgt 99 %.

## Patentansprüche

1. Mittel, enthaltend ein Cyclodepsipeptid der Formel 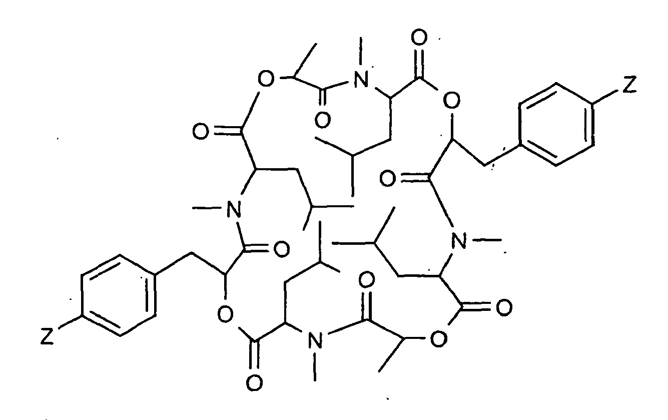 in welcher
Z für N-Morpholinyl, Amino, Mono- oder Dimethylamino steht,
allein oder in Mischung mit anderen Wirkstoffen, **dadurch gekennzeichnet, dass** die Mittel 1 ,2-Isopropylidenglycerin enthalten und diese Mittel zur topischen Anwendung bei Tieren geeignet sind.

2. Mittel gemäß Anspruch 1, enthaltend das Cyclodepsipeptid, in dessen Formel beide Reste Z für N-Morpholinyl stehen.

3. Mittel gemäß Anspruch 1 oder 2, **dadurch gekennzeichnet, dass** sie zusätzlich Benzylalkohol und/oder Propylenglykoldiacetat enthalten.

4. Mittel gemäß Anspruch 1 oder 2, **dadurch gekennzeichnet, dass** sie Benzylalkohol und Propylenglykoldiacetat enthalten.

5. Mittel gemäß einem der vorstehenden Ansprüche, enthaltend einen weiteren Wirkstoff, ausgewählt aus: L-2,3,5,6-Tetrahydro-6-phenylimidazothiazol, Benzimidazolcarbamaten wie Febantel, Pyrantel, Praziquantel und Ivermectin.

6. Verfahren zur Herstellung der Mittel gemäß einem der Ansprüche 1 bis 5, **dadurch gekennzeichnet, dass** man die Wirkstoffe mit den Lösungsmitteln und gegebenenfalls weiteren Hilfsstoffen vermischt.

7. Verwendung eines Cyclodepsipeptids der Formel 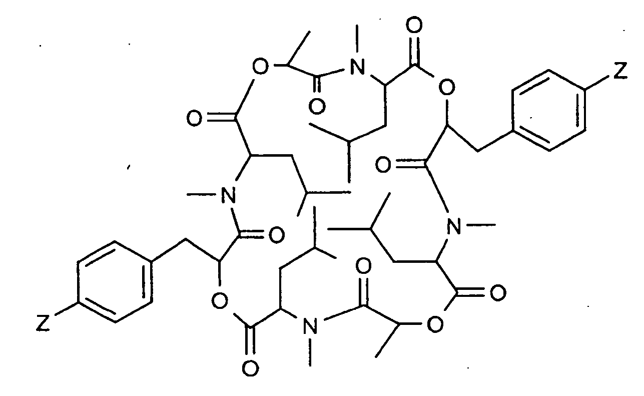 in welcher Z für N-Morpholinyl, Amino, Mono- oder Dimethylamino steht, zur Herstellung von Mitteln gemäß einem der Ansprüche 1 bis 5 zur Bekämpfung von Endoparasiten.

## Claims

1. Compositions, comprising a cyclodepsipeptide of the formula 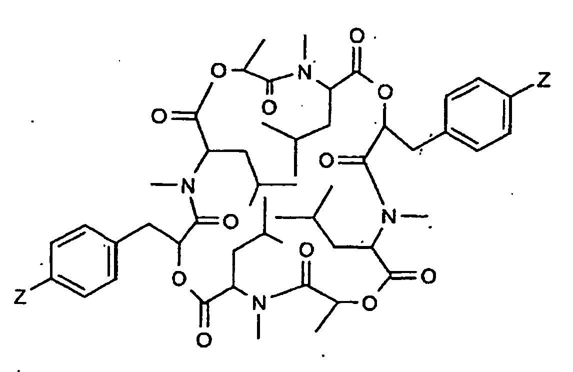 in which
Z represents N-morpholinyl, amino, mono- or dimethylamino,
on their own or as a mixture with other active compounds, **characterized in that** the compositions comprise 1,2-isopropylideneglycerol and that these compositions are suitable for topical administration in animals.

2. Compositions according to Claim 1, comprising the cyclodepsipeptide in whose formula both radicals Z represent N-morpholinyl.

3. Compositions according to Claim 1 or 2, **characterized in that** they additionally comprise benzyl alcohol and/or propylene glycol diacetate.

4. Compositions according to Claim 1 or 2, **characterized in that** they comprise benzyl alcohol and propylene glycol diacetate.

5. Compositions according to any of the preceding claims, comprising a further active compound selected from the group consisting of: L-2,3,5,6-tetrahydro-6-phenylimidazothiazole, benzimidazolecarbamates, such as febantel, pyrantel, praziquantel and ivermectin.

6. Process for preparing the compositions according to any of Claims 1 to 5, **characterized in that** the active compounds are mixed with the solvents and, if appropriate, further auxiliaries.

7. Use of a cyclodepsipeptide of the formula 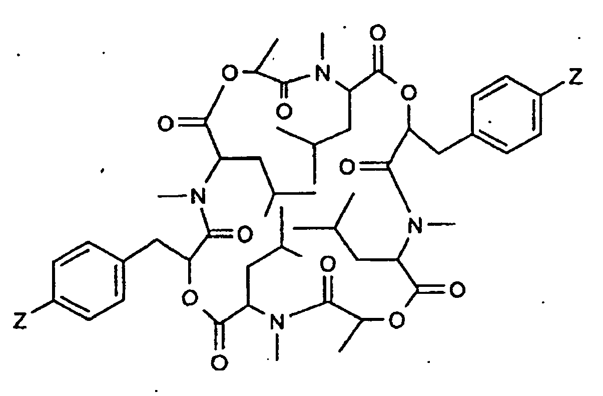 in which Z represents N-morpholinyl, amino, mono- or dimethylamino for preparing compositions according to any of Claims 1 to 5 for controlling endoparasites.

## Revendications

1. Agents contenant un cyclodepsipeptide de la formule : 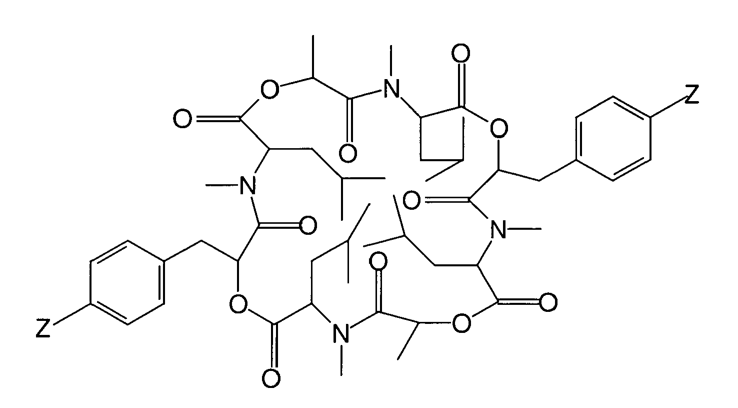 dans laquelle :
Z représente un radical N-morpholinyle, amino, mono- ou diméthylamino,
seul ou en mélange avec d'autres agents actifs, **caractérisés en ce que** les agents contiennent la 1,2-isopropylidèneglycérine et **en ce que** ces agents sont appropriés pour une application topique sur les animaux.

2. Agents suivant la revendication 1, contenant le cyclodepsipeptide, dans la formule duquel les deux restes Z représentent N-morpholinyle.

3. Agents suivant la revendication 1 ou 2, **caractérisés en ce qu'**ils contiennent en outre, l'alcool benzylique et/ou le diacétate de propylèneglycol.

4. Agents suivant la revendication 1 ou 2, **caractérisés en ce qu'**ils contiennent l'alcool benzylique et/ou le diacétate de propylèneglycol.

5. Agents suivant l'une des revendications précédentes, contenant un autre agent actif, choisi parmi : le L-2,3,5,6-tétrahydro-6-phénylimidazothiazole, des carbamates de benzimidazole comme le febantel, le pyrantel, le praziquantel et l'ivermectine.

6. Procédé de préparation de l'agent suivant une des revendications 1 à 5, **caractérisé en ce que** l'on mélange les agents actifs avec les solvants et le cas échéant, d'autres auxiliaires.

7. Utilisation d'un cyclodepsipeptide de la formule : 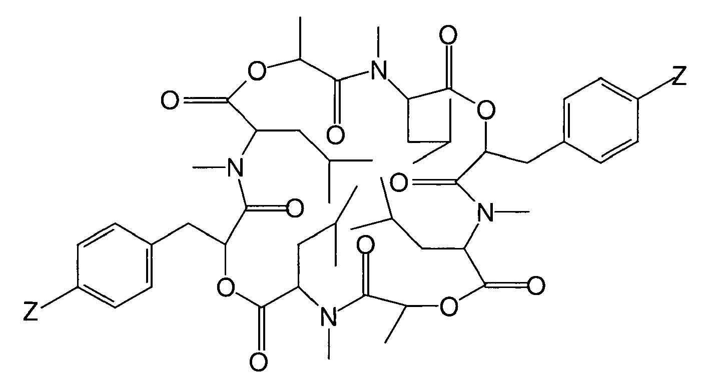 dans laquelle :
Z représente un radical N-morpholinyle, amino, mono- ou diméthylamino,
pour la préparation d'agents selon une des revendications 1 à 5 pour lutter contre les endoparasites.
